(19) **Europäisches Patentamt**
**European Patent Office**
**Office européen des brevets**

(11) **EP 4 756 038 A1**

(12) **EUROPEAN PATENT APPLICATION**
published in accordance with Art. 153(4) EPC

(43) Date of publication:
**10.06.2026 Bulletin 2026/24**

(51) International Patent Classification (IPC):
*C12Q 1/6883* (2018.01)    *C12Q 1/25* (2006.01)

(21) Application number: **24848436.2**

(22) Date of filing: **29.07.2024**

(86) International application number:
**PCT/ES2024/070483**

(87) International publication number:
**WO 2025/027224 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.07.2023 ES 202330668**

(71) Applicants:
• **Servicio Andaluz de Salud**
**41071 Sevilla (ES)**
• **Consorcio Centro de Investigación Biomédica en
Red**
**28029 Madrid (ES)**

(72) Inventors:
• **GARCÍA SERRANO, Sara**
**28029 Madrid (ES)**

• **RUIZ DE ADANA, María Soledad**
**41071 Sevilla (ES)**
• **GÓMEZ ZUMAQUERO, Juan Miguel**
**29590 Málaga (ES)**
• **GARCÍA ESCOBAR, Eva**
**28029 Madrid (ES)**
• **LAGO SAMPEDRO, Ana María**
**29590 Málaga (ES)**
• **ROJO MARTÍNEZ, Gemma**
**41071 Sevilla (ES)**

(74) Representative: **San Martin Alarcia, Esther**
**C/ Zumaia 33A, 1º b**
**48007 Bilbao (ES)**

Remarks:
The complete document including Reference
Table(s) and the Sequence Listing(s) can be
downloaded from the EPO website

(54) **BIOMARKERS AND METHOD FOR DIAGNOSING MONOGENIC DIABETES IN YOUNG
ADULTS CARRYING DELETERIOUS HNF1A ALLELES**

(57)    The present invention relates to the *in vitro* use of the expression levels of SEQ ID NO.: 3 (miR-19a-3p), SEQ ID NO.: 6 (miR-16-5p) and ultrasensitive CRP (hs-CRP) as biomarkers for diagnosing non-autoimmune monogenic maturity-onset diabetes of the young in individuals carrying deleterious HNF1A alleles (HNF1A-MODY). The invention further relates to an associated diagnostic method and a kit for carrying out said method.

EP 4 756 038 A1

**Description**

**FIELD OF THE INVENTION**

The invention relates to the field of medicine, more specifically to the field of medical prognosis and diagnosis, and even more specifically relates to the use of miRNAs and ultrasensitive CRP (hs-CRP or us-CRP) for the prognosis and diagnosis of monogenic Maturity Onset Diabetes of the Young adults carrying deleterious HNF1A alleles.

**STATE OF THE ART**

Although several genes are involved in monogenic diabetes *"MODY"* (from the acronym Maturity Onset Diabetes of the Young), "MODY HNF1A" (hepatocyte nuclear factor -1 alpha) is the most common form in adults and has a significant effect on treatment when properly diagnosed. Common clinical criteria for selecting individuals for MODY genetic testing include the onset of diabetes before age 25, preserved endogenous insulin production, absence of pancreatic autoimmunity, and consecutive generations of diabetes. These criteria clearly overlap with the characteristics of type 1 diabetes (T1DM) and type 2 diabetes (T2DM), so many people with MODY HNF1A remain unrecognized, especially if they do not meet the classic criteria for MODY. In this document, we define MODY HNF1A as non-autoimmune diabetes with onset in young adults in individuals carrying deleterious HNF1A alleles.

Due to its low frequency, monogenic diabetes is not usually the first diagnosis in a subject with hyperglycemia, and furthermore, many countries have limited access to genetic testing for its determination. Establishing a correct molecular diagnosis of MODY HNF1A allows the treatment to be changed to sulfonylureas or glinides, which can provide excellent diabetes control for decades. Correct diagnosis also facilitates rapid identification of affected family members and in the future, planning emerging gene therapy strategies.

Criteria such as the absence of Beta-cell autoantibodies and the presence of C-peptide exclude most cases of autoimmune diabetes or T1DM. Work in this area has mainly focused on the discrimination of MODY that occurs in childhood by selecting beta cell antibody-negative children for further investigation. A recent study took a similar approach with young adults diagnosed before age 30, although most had a clinical label of T1DM.

The differentiation of patients with T2DM, particularly in an older group where the proportion of MODY is lower, is more complex. The addition of MODY HNF1A specific biomarkers based on extrapancreatic manifestations of HNF1A could aid in the differentiation of this type of non-autoimmune diabetes.

The HNF1A gene encodes a transcription factor that regulates the expression of many genes. The expression of C-reactive protein (CRP) in the liver is regulated by HNF1A, and genome-wide association studies (GWAS) showed that plasma CRP levels were associated with genetic variation near HNF1A. A study conducted by the Owens group (Juszczak et al., 2019) reported that hs-CRP levels were lower in subjects with HNF1A MODY compared to other forms of diabetes, and especially discriminating in those with T2DM onset in young adults.

The use of biomarkers is increasing, and technical advances allow a more accurate identification of the processes involved in pathological progression. Recently, it has been shown that alternative biomarkers, such as circulating microRNAs *(miRNAs),* are associated with T2DM along with many other diseases (La Sala, Micheloni, De Nigris, Prattichizzo, & Ceriello, 2018; Prattichizzo et al., 2016; Zampetaki et al., 2010). miRNAs are small endogenous non-coding RNAs approximately 18-22 nucleotides in length, capable of modulating the expression of complementary messenger RNAs by pairing with the untranslated region (3'-UTR) (DP, 2004). They are also known to be involved in the regulation of most molecular processes. There is consistent scientific evidence regarding the relationship between miRNAs and metabolic processes, including insulin metabolism and glucose homeostasis (Kato, Castro, & Natarajan, 2013) . Various studies show how miRNAs alter gene expression in insulin-producing and insulin-sensitive tissues; examples include the pancreas regulated by miR-375 (Poy et al., 2004) and the liver by miR-122 (Esau et al., 2006). Circulating miRNAs have been detected in the bloodstream, where they exhibit high stability and reproducibility. Circulating miRNAs can provide clinical information about pathophysiological conditions, suggesting their important role in the pathogenesis, early diagnosis, and progression of diabetes.

Although the precise mechanisms of miRNA release into the bloodstream are only partially understood, it seems that miRNAs reach the circulatory system through a complex mechanism of release from cells with extracellular vesicles (EVs) or carrier proteins (de Candia, Torri, Pagani, & Abrignani, 2014). Circulating miRNAs are candidates as new biomarkers of insulin resistance and adiposity (Corona-Meraz et al., 2019), as well as for monitoring response to therapy with respect to glycemic targets (Catanzaro et al., 2018; Nunez Lopez, Retnakaran, Zinman, Pratley, & Seyhan, 2019; Yang et al., 2017) and for diabetes complications (Chien et al., 2015; Spinetti et al., 2013), but their involvement in the development of monogenic diabetes is unknown.

The objective of the present invention has been to develop a method based on circulating miRNAs which, together with hs-CRP, whose low levels have been described in MODY HNF1A, is useful in the differential diagnosis between patients with T2DM and MODY HNF1A. In particular, the value of different miRNAs was evaluated, which were selected from the literature for their involvement in insulin secretion and beta cell and pancreatic islet regeneration. Of these, mir-19 and mir-16-5p were finally selected, together with hs-CRP, as biomarkers to develop an algorithm that will distinguish subjects with MODY HNF1A from subjects with T2DM, as this is the most common clinical dilemma in the care of diabetes with C-peptide present and negative autoimmunity in adults.

## DESCRIPTION OF THE INVENTION

A microRNA (abbreviated as "miRNA") is a small non-coding RNA molecule found in both plants and animals, and which often plays a role in the post-transcriptional regulation of gene expression.

MicroRNAs contain approximately 22 nucleotides (approx. 18-25 nucleotides) and are single-stranded non-coding RNAs with a certain secondary structure that negatively regulate (inhibit) gene expression either by inhibiting translation or by cleaving messenger RNA (mRNA). Therefore, miRNAs are post-transcriptional regulators that bind to complementary sequences of mRNA transcripts, which generally results in repression of translation or degradation of the target, leading to gene silencing. In one particular aspect of the invention, understanding the molecular details of the action of the miRNAs of the invention is not critical, since the detected levels of the indicative miRNAs allow the methods of the invention to be performed.

Under a standard nomenclature system, names are assigned to the experimentally confirmed miRNAs as follows: the prefix "mir" is followed (by a hyphen and) a number, whereby the latter may indicate the order of the names. Uncapitalized "mir" refers to pre-miRNA, while a capital "miR -" refers to the mature form miRNAs with nearly identical sequences, except one or two nucleotides, are annotated with an additional lowercase letter, e.g., miR-99a. Pre-miRNAs leading to 100% identical mature miRNAs but which are located at different places in the genome are indicated with an additional hyphenated number suffix. The species of origin can be designated with a three-letter prefix, e.g., hsa-miR-223 is a human *(Homo sapiens)* miRNA. Since, in the context of this document, all individualized miRNAs are human miRNAs, the prefix "hsa-" is sometimes omitted. When two mature miRNAs originate from opposite arms of the same pre-miRNA, they are denoted with a suffix -3p or -5p, such as miR-142-3p. When relative expression levels are known, an asterisk after the name indicates a miRNA expressed at low levels relative to the miRNA on the opposite arm of a hairpin. Most known miRNA genes are located in intergenic regions or oriented antisense to neighboring genes and are therefore believed to be transcribed as independent units. Their genes are generally transcribed by RNA polymerase II, and the transcripts are processed, exported from the nucleus, and further processed by specific mechanisms, as is well known in the art (see, e.g., He et al., Nat. Rev. Genet. 2004 Jul; 5 (7): 522-31). miRNA sequences can be accessed at http://www.mirbase.org.

Thus, the term "miRNA of the invention" will be used to refer to any miRNA selected from the group consisting of mir-181, mir-15, mir-19a-3p, mir-24, mir-375, mir-16-5p, and mir-124-3p.

**Table** 1: miRNA sequences of the invention.

| Name | SEQ ID No. | Sequence (5'→ 3') | Accession number |
|---|---|---|---|
| hsa-miR-181a-5p | 1 | AACAUUCAACGCUGUCGGUGAGU | MIMAT0000256 |
| hsa-miR-15b-5p | 2 | UAGCAGCACAUCAUGGUUUACA | MIMAT0000417 |
| hsa-miR-19a-3p | 3 | UGUGCAAAUCUAUGCAAAACUGA | MIMAT0000073 |
| hsa-miR-24-3p | 4 | UGGCUCAGUUCAGCAGGAACAG | MIMAT0000080 |
| hsa-mir-375 | 5 | CCCCGCGACGAGCCCCUCGCACA AACCGGACCUGAGCGUUUUGUUC GUUCGGCUCGCGUGAGGC | MI0000783 |
| hsa-mir-16-5p | 6 | UAGCAGCACGUAAAUAUUGGCG | MIMAT0000069 |
| hsa-miR-124-3p | 7 | UAAGGCACGCGGUGAAUGCCAA | MIMAT0000422 |

A first aspect of the invention relates to the *in vitro* use of the expression levels of SEQ ID NO: 3 (miR-19a-3p), SEQ ID NO: 6 (miR-16-5p) and ultrasensitive CRP (hs-CRP) as biomarkers for predicting and/or diagnosing non-autoimmune monogenic maturity onset diabetes of the young in individuals carrying deleterious HNF1A alleles (MODY HNF1A).

In a preferred embodiment, the use of SEQ ID NO: 3 (miR-19a-3p), SEQ ID NO: 6 (miR-16-5p) and ultrasensitive CRP (hs-CRP) is simultaneous. In another preferred embodiment, the expression levels of one or more of the following miRNAs are also used as biomarkers: SEQ ID NO: 1 (miR-181); SEQ ID NO: 2 (miR-15); SEQ ID NO: 4 (miR-24), SEQ ID NO: 5 (miR-375) and SEQ ID NO: 7 (miR-124).

Another aspect of the invention relates to a biomarker expression profile suitable for diagnosing non-autoimmune monogenic maturity onset diabetes of the young in individuals carrying deleterious HNF1A alleles (MODY HNF1A), hereinafter referred to as the biomarker expression profile of the invention, comprising the expression levels of SEQ ID NO: 3 (miR-19a-3p), SEQ ID NO: 6 (miR-16-5p) and ultrasensitive CRP (hs-CRP). In another preferred embodiment, the biomarker expression profile of the invention further comprises expression levels of one or more of the miRNAs as set forth in the following SEQ ID NOs: SEQ ID NO: 1 (miR-181); SEQ ID NO: 2 (miR-15); SEQ ID NO: 4 (miR-24); SEQ ID NO: 5 (miR-375) and SEQ ID NO: 7 (miR-124).

METHOD FOR THE DIAGNOSIS/PROGNOSIS OF THE INVENTION

Another aspect of the invention relates to an *in vitro* method for diagnosing and/or prognosing non-autoimmune monogenic maturity onset diabetes of the young in individuals carrying deleterious HNF1A alleles, comprising:

a) measuring the expression levels of SEQ ID NO: 3 (miR-19a-3p), SEQ ID NO: 6 (miR-16-5p), and ultrasensitive CRP (hs-CRP) in a biological sample,

b) applying the formula

$$P=1/(1 + e^{-(0.788 - 2.646*hs\text{-}CRP + 4.630*miR\text{-}19\text{-}3p - 3.246*miR\text{-}16\text{-}5p)})$$

where the variables have been calculated as follows: to approximate them to a normal distribution, $\log_{10}$ and the Z-score ($Z \sim N(0.1)$) were applied to standardize them and enable comparison between them.

$$hs\text{-}CRP = Z\ score\ \log_{10}\ hs\text{-}CRP$$

$$miR\text{-}19a\text{-}3p = Z\ score\ \log_{10}\ 2^{deltaCtmiR\text{-}19a\text{-}3p}$$

$$miR\text{-}16\text{-}5p = Z\ score\ \log_{10}\ 2^{deltaCtmiR\text{-}16\text{-}5p}$$

c) classifying the individual in the group of individuals suffering from MODY HNF1A when they have a P greater than or equal to 0.311, more preferably greater than or equal to 0.40, and much more preferably greater than or equal to 0.83.

The method of the present invention allows individuals with P values < 0.05 to be classified with certainty in the group of individuals who do not suffer from MODY HNF1A.
The first method of the invention involves comparing said miRNA levels with the levels of said miRNAs in a reference sample or with a median value. In the context of the present invention, "reference sample" means the sample used to determine the variation in miRNA expression levels in the present invention. In a preferred embodiment, the reference value is obtained from the expression values obtained from a sample with individuals who do not have the disease, i.e., who do not suffer from MODY HNF1A.
Preferably, reference samples are taken from several individuals who do not have the disease and are combined, so that the reference value reflects the average value of these molecules in the population of individuals not suffering from MODY HNF1A. "Reference value" is the expression level of a miRNA of the invention in a reference sample.
In a preferred embodiment, expression levels are normalized with respect to the control miRNA, in order to mitigate expression variations of non-biological origin. In a more preferred embodiment, the control miRNA is the so-called spike-in template UniSp6, a synthetic miRNA molecule that we introduce when performing RT with the miRCURY LNATM Universal RT kit from Qiagen (ref 339340) following the manufacturer's protocol and recommendations. As a standardization method, RTs were also performed from total RNA concentrations of 25 ng (measured in nanodrop).

**Table 2:** Control miRNA: Spike-in UniSp6.

| Name | SEQ ID No. | Sequence (5'→ 3') | Qiagen Cat. No. |
|---|---|---|---|
| Spike-in UniSp6 | 8 | Sequence not provided by the manufacturer | 339340 |

In a preferred embodiment of this aspect of the invention, the biological sample is selected from blood, plasma and serum. In an even more preferred embodiment the biological sample is serum.

In another preferred embodiment of this aspect of the invention, the result can be obtained by any of the following techniques: (i) a method of generating miRNA profiles, such as a microarray, and/or (ii) a method comprising PCR (polymerase chain reaction), such as real-time PCR and/or digital PCR. (iii) immunoassay.

In the invention, the method for determining the result, i.e., the expression level of miRNA, need not be particularly limited, and may be selected from a method of miRNA profiling, such as a microarray, and/or a method comprising PCR (polymerase chain reaction), such as real-time PCR and/or immunoassay; Northern Blot.

Real-time quantitative PCR (polymerase chain reaction) (generally abbreviated as RQ-PCR, RT-qPCR, rt-PCR or qPCR) is a sensitive and reproducible technique for quantifying of miRNA expression that can be used particularly for profiling miRNA expression in cells and tissues. Any method of evaluating RT-PCR results may be used, and the $\Delta Ct$ method and $\Delta\Delta Ct$ method may be preferred. The $\Delta\Delta Ct$ method is described in detail by Livak et al. (Methods 2001, 25: 402-408). (Ct = Cycle threshold values). In implementing the present invention, the $\Delta\Delta Ct$ method described by Livak et al. (Methods 2001, 25: 402-408) will be used preferentially. The $\Delta\Delta Ct$ method will include a 'control sample' and a 'subject sample'. The 'subject sample' is a sample from the subject to be analyzed. For each sample, a target miRNA (here: miRNA of interest) and a synthetic and/or endogenous control miRNA (as described below) are included for PCR amplification from aliquots (typically in series). Typically, several replicates are used for each diluted concentration to derive the amplification efficiency. PCR amplification efficiency can be defined as the amplification percentage (from 0 to 1). During the qPCR reaction, software typically measures for each sample the cycle number at which the fluorescence (PCR amplification indicator) crosses an arbitrary line, the threshold. This crossing point is the Ct value.

A microarray is an array on a solid substrate (usually a glass slide or a thin silicon film cell) that analyzes large amounts of biological material, in this case a large number of different miRNAs or, preferably, their reverse DNA transcripts, which are detectable by specific probes immobilized on the solid substrate.

A Northern blot involves the use of electrophoresis to separate RNA samples by size and subsequent detection with a hybridization probe complementary to (part of) the target sequence of the RNA of interest.

The method of the present invention can be applied to samples from individuals of any sex, i.e., male or female, and at any age.

In the method of the present invention, miRNA expression can be normalized, preferably relative to the expression of another RNA molecule. There are well-known normalization methods in the prior art, although to date there is no consensus or universal method for the standardization of miRNAs in serum.

For the development of the algorithm, the control miRNA called spike-in template UniSp6 has been used, as it is a method that allows us to use it in other groups of subjects and ensures the replication of measurements in different populations.

The invention provides a method for assigning a human subject to one of two groups: group 1, comprising subjects identifiable by the method of the invention, and group 2, representing the remaining subjects.

It is possible to provide personalized therapy to an individual depending on whether the individual is assigned to group 1 or group 2. Group 1 comprises subjects identifiable by the method of the invention as individuals suffering from MODY HNF1A, and group 2 represents the remaining subjects.

KIT OR DEVICE OF THE INVENTION

Another aspect of the invention relates to a kit or device comprising at least one or more oligonucleotides capable of hybridizing with miRNAs established as SEQ ID NO: 3 and 6 under stringent conditions.

It is preferred that said oligonucleotide(s) to be capable of doing so under stringent conditions. In a preferred embodiment, one or more of said one or more oligonucleotides (preferably DNA) are further defined by the following probes or primers: hsa-miRXX-miRCURY LNA miRNA measured with syber, specific LNA™ PCR primers.

More preferably the kit also comprises oligonucleotide(s) capable of hybridizing with miRNAs established as SEQ ID NO: 1, 2, 4, 5, and/or 7 under stringent conditions.

Astringency is a term used in hybridization experiments. Astringency reflects the degree of complementarity between the oligonucleotide and the nucleic acid (which in this case is the miRNA to be detected); the higher the astringency, the higher the percentage of homology between the probe and the nucleic acid bound to the filter. The skilled person knows well that the temperature and salt concentrations have a direct effect on the results obtained. It is recognized that the hybridization results are related to the number of degrees below the Tm (melting temperature) of the DNA in which the experiment is performed. Often, stringent conditions are defined as a wash with 0.1X SSC (saline-sodium citrate (SSC) buffer at 65°C (SSC is usually provided as a 20X stock solution, consisting of 3 M sodium chloride and 300 mM trisodium citrate (adjusted to pH 7.0 with HCl)).

In particular embodiments, the kit is selected from (a) a kit suitable for PCR, (b) a kit suitable for Northern Blot (c) Immunoassay, and (d) a kit suitable for microarray analysis. Two or more of these embodiments may also be combined, such that the kit may comprise, for example, both (a) and (c).

In the case of (a) a kit suitable for PCR, this PCR is typically real-time quantitative PCR (RQ-PCR), a sensitive and reproducible gene expression quantification technique. In this case, it is desirable that the kit further comprises a polyT oligonucleotide primer in addition to the oligonucleotide(s) of the kit. The polyT oligonucleotide primer can be used in conjunction with the oligonucleotide(s) of the invention for priming PCR, after polyadenylation of the isolated miRNAs by methods known to those skilled in the art, such as the use of poly-(A) polymerase and ATP. These reagents may optionally be included in the kit.

A Northern blot (b) involves the use of electrophoresis to separate RNA samples by size and subsequent detection with an oligonucleotide (s) (hybridization probe) complementary to (part of) the target RNA sequence of interest.

The kit may also be an immunoassay type (c), with controls, calibrators, and a standard curve to measure miRNA expression in a spectrophotometer.

It is also possible for the oligonucleotide(s) to be immobilized in spots on a surface (preferably solid). In one embodiment, the kit comprises a microarray (d). An RNA microarray is an array on a solid substrate (usually a glass slide or a thin silicon film cell) that analyzes large amounts of different RNAs (in this case, miRNAs), which can be detected by specific probes immobilized at points on the solid substrate. Each spot contains a specific nucleic acid sequence, typically a DNA sequence, known as probes (or informers). While the number of spots is not limited, there is a preferred embodiment in which the microarray is adapted to the methods of the invention. In one embodiment, a customized microarray of this type comprises fifty spots or less, such as thirty spots or less, including twenty spots or less.

The kit or device of the invention may be used and the use is not particularly limited, although use in the method of the invention in any of its embodiments is preferred.

In another preferred embodiment of this aspect of the invention, the oligonucleotides, primers, probes, or antibodies are modified or labeled, for example, but not limited to, by radioactive or immunological labeling. Thus, preferably, the oligonucleotides exhibit modifications at any of their nucleotides, such as, but not limited to, nucleotides having any of their atoms with a radioactive isotope, typically $^{32}$P or tritium, immunologically labeled nucleotides, such as with a digoxigenin molecule, and/or immobilized on a membrane. Several possibilities are known in the prior art.

The kit or device of the invention may comprise controls, program instructions and information necessary to carry out any of the methods of the invention. Another aspect of the invention relates to the use of the kit according to any of the embodiments described above to carry out any of the embodiments of the method of the invention.

## AUTOMATION OF THE METHODS OF THE INVENTION

The invention also extends to computer programs adapted so that any processing means can carry out the methods of the invention. Such programs may be in the form of source code, object code, an intermediate source code and object code, for example, as in partially compiled form, or in any other form suitable for use in implementing the processes according to the invention. Computer programs also cover cloud-based applications based on said method.

In particular, the invention encompasses computer programs arranged on or within a carrier. The carrier may be any entity or device capable of supporting the program. When the program is embodied in a signal that can be transported directly by a cable or other device or means, the carrier may consist of said cable or other device or means. As a variant, the carrier could be an integrated circuit in which the program is included, the integrated circuit being adapted to execute, or to be used in the execution of, the corresponding processes.

For example, the programs could be incorporated into a storage medium, such as a ROM memory, a CD ROM memory or a semiconductor ROM memory, a USB memory, or a magnetic recording medium, for example, a floppy disk or a hard disk. Alternatively, the programs could be supported on a transmissible carrier signal. For example, it could be an electrical or optical signal that could be carried via electrical or optical cable, radio, or any other means.

The invention also extends to computer programs adapted so that any processing medium can implement the methods of the invention. Such programs may take the form of source code, object code, an intermediate source code and object code, for example, as partially compiled, or in any other form suitable for use in implementing the processes according to the invention. The computer programs also encompass cloud-based applications based on said procedure.

Therefore, another aspect of the invention relates to a computer-readable storage medium comprising program instructions capable of causing a computer to perform the steps of any of the methods of the invention.

Another aspect of the invention relates to a transmissible signal comprising program instructions capable of causing a computer to perform the steps of any of the methods of the invention.

Specifically, a calculator has been developed with the formula of the algorithm of the invention

$$P = 1/(1 + e^{-(0.788 - 2.646*hs\text{-}CRP + 4.630*miR\text{-}19\text{-}3p - 3.246*miR\text{-}16\text{-}5p)})$$

to facilitate the calculation of probability, using programming code and a user-friendly interface.

The diagnostic calculator was created using the Python programming language and the Tkinter library. This library provides different tools for creating and developing a graphical user interface and allows the interface to be compatible with most operating systems. The interface consists of an application window where the different "widgets" that make up the "calculator" are distributed. It includes:

- 4 text boxes in which the values of the features that make up the algorithm are entered.

- 1 button which performs the function for calculation in the equation obtained in the development of the algorithm.

- 1 text box wherein the result of the calculation is displayed and the probability of suffering the disease in 4 levels: negative, possible positive, probable positive and very likely positive; according to the value of p is less than 0.311 (negative), greater than or equal to 0.311 (possible positive), greater than or equal to 0.40 (probable positive), or greater than or equal to 0.83 (very probable positive).

An "isolated biological sample" includes, but is not limited to, cells, tissues, and/or biological fluids from an organism, obtained by any method known to one skilled in the art. Preferably, the isolated biological sample is peripheral venous blood.

The term "individual", as used in the description, refers to animals, preferably mammals, and more preferably, humans. The terms "individual", "human subject", and "subject" are used interchangeably herein and are synonymous with "patient" and are not intended to be limiting in any respect, as the subject may be of any age, sex, and physical condition. The terms "one or more", "at least one", or "several" as used in this document, include one and the individualized specification of any number that is more than one, such as two, three, four, five, six, etc. The term " comprises" may also be interpreted, in a particular embodiment, as "consisting of". The term "comprising" and its variants are not intended to exclude other technical features, additives, components, or steps. For those skilled in the art, other objects, advantages, and features of the invention will be apparent in part from the description and in part from the practice of the invention.

## DESCRIPTION OF THE FIGURES

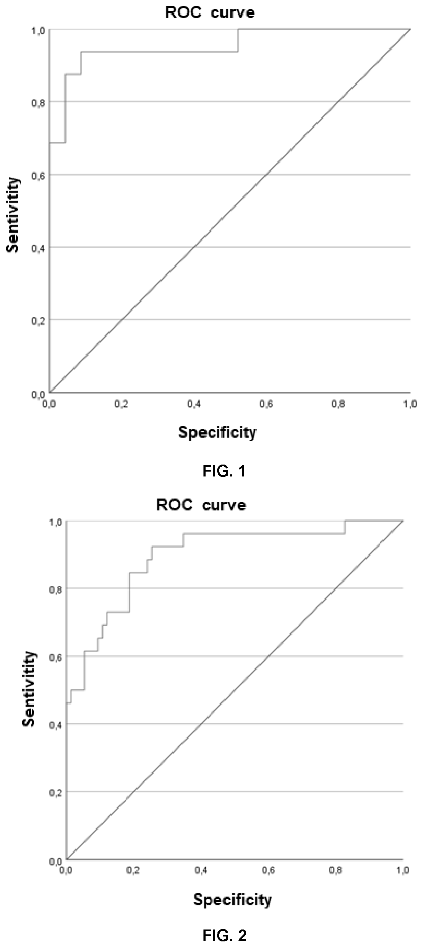

**Figure 1:** ROC curve of the model for the design of the algorithm. Statistical method for determining the diagnostic accuracy of the method of the invention, in particular its sensitivity and specificity and its discriminatory capacity, the area under the curve of the model, the probability that, given a pair of individuals, one sick and the other healthy, the test will classify them correctly.

**Figure 2:** ROC curve of the algorithm model with samples for validation.

## EXAMPLES OF THE INVENTION

### Participants in the study for algorithm design.

MODY HNF1A subjects were recruited from the Diabetes Unit of the Regional University Hospital of Malaga (HRUM) and subjects with T2DM were selected from the studydi@bet.es. They were matched by age, BMI, and sex. Number of MODY HNF1A patients = 17 with previous diagnosis made by Sanger. Number of subjects with T2DM = 22.

### Participants in the study for algorithm validation.

MODY HNF1A subjects were recruited from the Diabetes Unit of the Regional University Hospital of Malaga (HRUM) and subjects with T2DM were selected from the studydi@bet.es. They were matched by age, BMI, and sex. Number of MODY HNF1A patients = 29 with previous diagnosis made by Sanger. Number of subjects with T2DM = 75

### DNA sequencing and evaluation of HNF1A alleles.

DNA was extracted, amplified, and sequenced using the Sanger method. Mutation Surveyor version 5.0.1 (SoftGenetics, Cambridge, UK) was used to detect variants compared to the reference sequence (NM_000545.5). Systematic assessment of rare HNF1A alleles (minor allele frequency - MAF- 1%) was subsequently performed and aligned with the American College of Medical Genetics (ACMG) classification. This included clinical features, co-segregation of the allele with diabetes in the family and *in silico* analysis of wrong-sense variants using sorting intolerant from tolerant (SIFT) classification, polymorphism phenotyping version 2 (PolyPhen2), and protein variation effect analyzer (PROVEAN). The potential effect on splicing was examined using Human Splicing Finder (HSF).

**Biochemical and immunological assays.**

hs-CRP was measured using a high-sensitivity, wide-range, latex-enhanced immunoturbidimetric assay on an ADVIA 2400 analyzer (Siemens Healthcare Diagnostics, Erlangen, Germany) with a limit of quantification of 0.01 mg/L. hs-CRP measurements for MODY HNF1A samples were performed using the Abbott hs-CRP method, and the lowest quantifiable level was 0.1 mg/L.

The methods were reproducible, with a coefficient of variation for both below 10.5% throughout the tested concentration range. Comparison of clinical samples measured by both methods using Passing and Bablock regression showed the agreement with the Abbott method = 0.26 + 0.99 (Siemens method).

**Methodology for determining miRNAs.**

We selected 10 candidate miRNAs from the literature as potential biomarkers for the diagnosis of MODY HNF1A based on criteria related to insulin synthesis and beta cell and pancreatic islet regeneration processes. We then conducted a pilot study with a group of patient serum samples in which these 10 candidate miRNAs were measured, and after this test we obtained consistent results regarding their determination in serum for the following 7: miR181, miR15, miR19-3p, miR124-3p, miR24, miR375, and miR16-5p.

miRNA extraction from serum samples was performed using automated methods on Promega Maxwell 16 (serial number 23627808) with Maxwell® 16 miRNA Tissue Kit (Promega Biotech Ibérica SL, Madrid, Spain) and converted to microcDNA by reverse transcription with Qiagen miRCURY LNATM Universal RT Kit (ref. 339340) following the manufacturer's recommendations for each kit. miRNA expression levels were measured by real-time qPCR in 384-well plates on a Light Cycler 480 (Roche Diagnostics, S.L., Barcelona, Spain) at the IBIMA Genomics Platform. The master mix was prepared following Exiqon guidelines with GoTaq (R) qPCR Master Mix (Promega Biotech Ibérica S.L., Madrid, Spain) and specific LNA™ PCR primer sets (Qiagen). miRNA analysis was performed using the delta Ct method.

For data normalization, negative controls and a specific calibrator were included in all plates for inter-plate normalization. Those miRNA determinations with Ct $\geq$ 38 were considered below the detection limit and were removed from the analysis.

**Statistical analysis.**

Subject characteristics and experimental results were analyzed using SPSS 23 software. Continuous data are presented as medians with interquartile range (IQR), and the Kruskal-Wallis test was used to compare groups. A p-value of 0.05 was considered statistically significant.

The diagnostic efficacy analysis was performed by comparing the two groups of patients by step-by-step logistic regression analysis with which the best diagnostic model was selected. The predicted probabilities of the model were dichotomized based on the best cut-off calculated by the ROC curve. The predictive values were then calculated based on the cut-off.

**Results.**

The miRNAs miR-181, miR-15, miR-19-3p, miR-24, miR 124-3p, miR-375, and miR-16-5p were measured along with hs-CRP in people with T2DM and MODY HNF1A. All showed significant differences when adjusted for age, sex, and BMI.

On a practical level, for proper phenotyping and activation of genetic testing for MODY HNF1A, we are interested in differentiating diabetes due to MODY HNF1A mutation from T2DM; therefore, diagnostic efficacy analysis was performed between these two groups by means of a step-by-step logistic regression analysis.

The final model includes the following variables: miR-19-3p, miR-16-5p, and hs-CRP, and is defined by the following algorithm:

$$P = 1/(1 + e^{-(0.788 - 2.646*hs\text{-}CRP + 4.630*miR\text{-}19\text{-}3p - 3.246*miR\text{-}16\text{-}5p)})$$

Therefore, this logarithm yields the probability of presenting MODY HNF1A (MODY 3) based on the three variables miR-19-3p, miR-16-5p, and hs-CRP, where the values of the analyzed variables were as follows:

Table 3: Description of the variables used to design the algorithm.

| | T2DM patients; n=23 | MODY 3 patients; n=17 | P Mann-Whitney |
|---|---|---|---|
| | Mean ± SD | Mean ± SD | |
| Ct miR19-3p | 33.38±1.9 | 31.13±1.76 | <0.001 |
| Ct miR16-5p | 26.54±1.5 | 25.45±1.78 | 0.085 |
| UniSp6 | 19.06±0.2 | 18.82±0.32 | 0.01 |
| hs-CRP(mgL) | 5.66±6.02 | 1.48±1.24 | <0.001 |

The predicted probabilities are dichotomized based on the best cut-off calculated according to the best sensitivity and specificity by the ROC curve = 0.311.

Predictive values were calculated using this cut-off. The Nagelkerke R2 of the model is 0.777, indicating that 77.7% of the variability in the dependent variable is explained by the model.

Values of p>0.837 indicate MODY HNF1A with certainty.

Values of p< 0.056 rule out MODY HNF1A with certainty.

Table 4: Contrast result variables: Predicted probability of MODY HNF1A vs. T2DM with the algorithm model.

| Area | Standard error [a] | Asymptotic significance[b] | 95% asymptotic confidence interval | |
|---|---|---|---|---|
| | | | Lower limit | Upper limit |
| .954 | .035 | .000 | .885 | 1,000 |
| a. Under the nonparametric assumption b. Null hypothesis: true area = 0.5 | | | | |

Table 5: Diagnostic test results with samples for algorithm design.

| | | True diagnosis or reference criterion | | |
|---|---|---|---|---|
| | | Positive | Negative | Total |
| Diagnostic test result | Positive | 15 | 1 | 16 |
| | Negative | 2 | 21 | 23 |
| | Total | 17 | 22 | 39 |
| Positive = MODY 3; Negative = T2DM | | | | |

Of the 16 subjects diagnosed with MODY HNF1a, the algorithm correctly classified 15, and of the 23 subjects with T2DM, 21 were correctly classified within their group using the algorithm.

Table 6: Characteristics of the diagnostic algorithm based on the samples used for its development.

| | | 95% CI Lower limit | 95% CI Upper limit |
|---|---|---|---|
| Prevalence of the disease | 43.59 | 28.19 | 60.23 |
| Correctly diagnosed patients | 92.31 | 78.03 | 97.99 |
| Sensitivity | 88.24 | 62.25 | 97.94 |
| Specificity | 95.45% | 75.12% | 99.76% |
| Positive predictive value | 93.75 | 67.71 | 99.67 |
| Negative predictive value | 91.30 | 70.49 | 98.48 |
| Positive likelihood ratio | 19.41 | 2.84 | 132.78 |
| Negative likelihood ratio | 0.12 | 0.03 | 0.45 |

**Table 7:** Change in predictive values based on the prevalence of the disease in the population undergoing differential diagnosis.

| | Prevalence of the disease | | | | |
|---|---|---|---|---|---|
| | 43.6% | 27.9% | 16.2% | 8.8% | 4.6% |
| Positive predictive value | 93.4% | 88.2% | 78.9% | 65.2% | 48.4% |
| Negative predictive value | 91.3% | 95.4% | 97.6% | 98.8% | 99.4% |

According to calculations, this algorithm allows for the diagnosis of a patient with MODY HNF1A with a positive predictive value of 93.4% in a specialized monogenic diabetes clinic where adequate phenotyping is performed to increase the pre-test probability of monogenic diabetes.

**Validation**

Next, the predicted probabilities were calculated with the new subjects selected for validation, dichotomizing according to the same cut-off used in the development of the algorithm (0.311), calculated from the best specificity and sensitivity values from the ROC curve obtained from the model.

**Table 8:** Description of the variables used for validation.

| | T2DM patients n=77 | MODY 3 patients n=26 | P Mann-Whitney |
|---|---|---|---|
| | Mean $\pm$ SD | Mean $\pm$ SD | |
| Ct miR19-3p | 31.65$\pm$1.65 | 32.30$\pm$2.4 | <0.001 |
| Ct miR16-5p | 26.03$\pm$1.41 | 27.22$\pm$3.2 | <0.001 |
| UniSp6 | 17.35$\pm$1.08 | 20.25$\pm$0.78 | 0.003 |
| hs-CRP (mg/L) | 4.34$\pm$6.64 | 1.12$\pm$0.86 | <0.001 |

**Table 9.** Predicted probability algorithm model with samples for validation.

| Area under the curve | | | | |
|---|---|---|---|---|
| Test outcome variables: PPMODY3 **CRP_miR19 and 16_model** | | | | |
| | | | 95% asymptotic confidence interval | |
| Area | Std. Error[a] | Asymptotic significance[b] | Lower limit | Upper limit |
| .895 | .038 | .000 | .821 | .970 |
| a. Under the nonparametric assumption b. Null hypothesis: true area = 0.5 | | | | |

**Table 10:** Diagnostic test results with validation samples.

| | | True diagnosis or reference criterion | | |
|---|---|---|---|---|
| | | Positive | Negative | Total |
| Diagnostic test result | Positive | 25 | 36 | **61** |
| | Negative | 1 | 39 | **40** |
| | Total | **26** | **75** | **101** |
| Positive = MODY 3; Negative = T2DM | | | | |

Of the 26 subjects selected for validation diagnosed with MODY HNF1a, the algorithm correctly classified 25, and of the 75 subjects with T2DM selected for validation, 39 were correctly classified within their group using the algorithm.

Table 11: Characteristics of the diagnostic algorithm based on the samples used for validation.

|  |  | 95% CI Lower limit | 95% CI Upper limit |
|---|---|---|---|
| Prevalence of the disease | 25.74% | 17.79% | 35.57% |
| Correctly diagnosed patients | 63.37% | 53.14% | 72.56% |
| Sensitivity | 96.15% | 78.42% | 99.80% |
| Specificity | 52.00% | 40.24% | 63.56% |
| Positive predictive value | 40.98% | 28.80% | 54.30% |
| Negative predictive value | 97.50% | 85.27% | 99.87% |
| Positive likelihood ratio | 2.00 | 1.56 | 2.57 |
| Negative likelihood ratio | 0.07 | 0.01 | 0.51 |

The diagnosis of MODY HNF1A is carried out using sequencing techniques such as SANGER technology, which are gene-by-gene studies, or using next-generation sequencing (NGS) technology, which allows many genes to be studied at once. Physicians often have limited access to these tests, but in other countries they have greater access to them. Biomarkers can still be useful as a risk estimate prior to MODY HNF1A genetic testing and can also provide information in the context of interpreting results. In other countries, including Spain, very few genetic tests are routinely available, so biomarkers may continue to play a role in identifying individuals at higher risk of MODY HNF1A for single-gene Sanger sequencing approaches.

The combination of circulating biomarkers such as hs-CRP and miRNAs open a new study route to develop more affordable diagnostic tests that are a cost-effective alternative to genetic tests required for diseases such as monogenic diabetes. In those situations where the availability of genetic tests is more limited in routine clinical practice, these biomarkers may serve a pre-screening for identification of subjects at high risk of presenting a type of MODY. Currently, genetic test is the first-line diagnostic test, however, in these cases, the use of these biomarkers can guide us regarding the pathogenicity of new, undescribed variants or those of uncertain significance.

In conclusion, after our study we found that biomarkers mir-19-3p, mir-16-5p, and hs-CRP could differentiate individuals with pathological HNF1A alleles from those subjects with T2DM. A diagnostic protocol that combines clinical features with biomarkers could improve the selection of subjects for genetic testing for MODY HNF1A, the most common form of monogenic diabetes in adults. The application of this algorithm could optimize resources for health systems as it would provide more accurate information when indicating genetic testing.

Table 12: Comparison of price and execution time between techniques.

| Description | Approximate total execution time |
|---|---|
| Sanger sequencing | 4.1 hours |
| NGS panels (32 genes) | 8.10 hours |
| 2 miRNAs | 4.5 hours |
| hs-CRP | 6 hours |

It is also interesting to note that both Sanger sequencing and massive sequencing (NGS) require much more expensive equipment than that needed for miRNA determination and/or hs-CRP. The measurement of a miRNA can be performed in a classic thermocycler for real-time PCR, and the determination of hs-CRP only requires an absorbance reader that can be found in any basic clinical analysis laboratory. Therefore, and as discussed previously herein, the performance of biomarker determinations for the application of this MODY HFN1a diagnostic algorithm would be more feasible in centers that have more limited resources and therefore much more modest equipment.

References

1. Catanzaro, G., Besharat, Z. M., Chiacchiarini, M., Abballe, L., Sabato, C., Vacca, A., Ferretti, E. (2018). Circulating MicroRNAs in elderly type 2 diabetic patients. International Journal of Endocrinology, 2018.
2. Chien, H. Y., Lee, T. P., Chen, C. Y., Chiu, Y. H., Lin, Y. C., Lee, L. S., & Li, W. C. (2015). Circulating microRNA as a diagnostic marker in populations with type 2 diabetes mellitus and diabetic complications. Journal of the Chinese Medical Association, Vol. 78.

3. Corona-Meraz, F. I., Vázquez-Del Mercado, M., Ortega, F. J., Ruiz-Quezada, S. L., Guzmán-Ornelas, M. O., & Navarro-Hernández, R. E. (2019). Ageing influences the relationship of circulating miR-33a and miR-33b levels with insulin resistance and adiposity. Diabetes and Vascular Disease Research, 16(3), 244-253.

4. de Candia, P., Torri, A., Pagani, M., & Abrignani, S. (2014). Serum microRNAs as biomarkers of human lymphocyte activation in health and disease. Frontiers in Immunology, Vol. 5.

5. DP, B. (2004). MicroRNAs : Genomics, Biogenesis, Mechanism, and Function.pdf. Cell.

6. Esau, C., Davis, S., Murray, S. F., Yu, X. X., Pandey, S. K., Pear, M., ... Monia, B. P. (2006). miR-122 regulation of lipid metabolism revealed by in vivo antisense targeting. Cell Metabolism, 3(2), 87-98.

7. Juszczak, A., Pavić, T., Vučković, F., Bennett, A. J., Shah, N., Medvidović, E. P., ...Gornik, O. (2019). Plasma fucosylated glycans and C-reactive protein as biomarkers of HNF1A-MODY in young adult-onset nonautoimmune diabetes. Diabetes Care, 42(1), 17-26.

8. Kato, M., Castro, N. E., & Natarajan, R. (2013). MicroRNAs: Potential mediators and biomarkers of diabetic complications. Free Radical Biology and Medicine, Vol. 64, pp. 85-94.

9. La Sala, L., Micheloni, S., De Nigris, V., Prattichizzo, F., & Ceriello, A. (2018, October 1). Novel insights into the regulation of miRNA transcriptional control: implications for T2D and related complications. Acta Diabetologica, Vol. 55, pp. 989-998.

10. Nunez Lopez, Y. O., Retnakaran, R., Zinman, B., Pratley, R. E., & Seyhan, A. A. (2019). Predicting and understanding the response to short-term intensive insulin therapy in people with early type 2 diabetes. Molecular Metabolism, 20, 63-78.

11. Poy, M. N., Eliasson, L., Krutzfeldt, J., Kuwajima, S., Ma, X., MacDonald, P. E.., ...Nature, 432(7014), 226-230.

12. Prattichizzo, F., Giuliani, A., De Nigris, V., Pujadas, G., Ceka, A., La Sala, L., ... Ceriello, A. (2016, September 1). Extracellular microRNAs and endothelial hyperglycaemic memory: a therapeutic opportunity? Diabetes, Obesity and Metabolism, Vol. 18, pp. 855-867.

13. Spinetti, G., Fortunato, O., Caporali, A., Shantikumar, S., Marchetti, M., Meloni, M., ...Circulation Research, 112(2), 335-346.

14. Yang, Z. M., Chen, L. H., Hong, M., Chen, Y. Y., Yang, X. R., Tang, S. M., ... Chen, W. W. (2017). Serum microRNA profiling and bioinformatics analysis of patients with type 2 diabetes mellitus in a Chinese population. Molecular Medicine Reports, 15(4).

15. Zampetaki, A., Kiechl, S., Drozdov, I., Willeit, P., Mayr, U., Prokopi, M., ...Circulation Research, 107(6), 810-817.

**Claims**

1. *In vitro* use of expression levels of SEQ ID NO: 3 (miR-19a-3p), SEQ ID NO: 6 (miR-16-5p) and ultrasensitive CRP (hs-CRP) as biomarkers for diagnosing non-autoimmune monogenic maturity onset diabetes of the young in individuals carrying deleterious HNF1A alleles (MODY HNF1A).

2. The use according to claim 1, which further comprises using as biomarkers the expression levels of one or more miRNAs to be selected from: SEQ ID NO: 1 (miR-181); SEQ ID NO: 2 (miR-15); SEQ ID NO: 5 (miR-375) and SEQ ID NO: 4 (miR-24).

3. An *in vitro* method for diagnosing non-autoimmune monogenic maturity onset diabetes of the young in individuals carrying deleterious HNF1A alleles comprising:

   a) measuring the expression levels of SEQ ID NO: 3 (miR-19a-3p), SEQ ID NO: 6 (miR-16-5p) and ultrasensitive CRP (hs-CRP) in a biological sample,
   b)

$$P=1/(1 + e^{-(0.788 - 2.646*hs\text{-}CRP+4.630*miR\text{-}19\text{-}3p- 3.246*miR\text{-}16\text{-}5p)})$$

   c) classifying the individual into the group of individuals suffering from MODY HNF1A when they have a P greater than or equal to 0.311, more preferably greater than or equal to 0.40, and much more preferably greater than or equal to 0.83.

4. The method according to the previous claim, wherein the biological sample is selected from blood, plasma, and serum.

5. The method according to any of claims 3-4, wherein the biological sample is serum.

6. The method according to any of claims 3-5, wherein said result can be obtained by:

   (i) a method of generating miRNA profiles, such as a microarray, and/or
   (ii) a method comprising PCR (polymerase chain reaction), such as real-time PCR; and/or
   (iii) immunoassay.

7. Use of a kit or device comprising at least one or more oligonucleotides capable of hybridizing with the miRNAs set forth as SEQ ID NO: 3 and 6 under stringent conditions in a method according to any of claims 3-6.

8. Use according to the preceding claim, **characterized in that** the kit also comprises oligonucleotide(s) capable of hybridizing with the miRNAs set forth as SEQ ID NO: 1, 2, 5, and/or 4 under stringent conditions.

ROC curve

FIG. 1

ROC curve

FIG. 2

# EP 4 756 038 A1

## INTERNATIONAL SEARCH REPORT

| International application No. |
| --- |
| PCT/ES2024/070483 |

### A. CLASSIFICATION OF SUBJECT MATTER

*C12Q1/6883* (2018.01)
*C12Q1/25* (2006.01)
According to International Patent Classification (IPC) or to both national classification and IPC

### B. FIELDS SEARCHED

Minimum documentation searched (classification system followed by classification symbols)
C12Q

Documentation searched other than minimum documentation to the extent that such documents are included in the fields searched

Electronic data base consulted during the international search (name of data base and, where practicable, search terms used)

EPODOC, INVENES, WPI, BIOSIS, MEDLINE, EMBASE, INSPEC, NPL, EMBL-EBI, INTERNET

### C. DOCUMENTS CONSIDERED TO BE RELEVANT

| Category* | Citation of document, with indication, where appropriate, of the relevant passages | Relevant to claim No. |
| --- | --- | --- |
| A | SZOPA, M. et al. A decision algorithm to identify patients with high probability of monogenic diabetes due to HNF1A mutations. Endocrine. Apr 2019, Vol. 64, No 1, pages 75-81, ISSN 1355-008X (print), ISSN 1559-0100 (electronic), <DOI: 10.1007/s12020-019-01863-7> the whole document. | 1-8 |
| A | BONNER, C. et al. Identification of circulating microRNAs in HNF1A-MODY carriers. Diabetologia. Aug 2013, Vol. 56, No 8, pages 1743-1751, ISSN 0012-186X (print) ISSN 1432-0428 (electronic), <DOI: 10.1007/s00125-013-2939-4> the whole document. | 1-8 |

☒ Further documents are listed in the continuation of Box C.  ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| Date of the actual completion of the international search | Date of mailing of the international search report |
| --- | --- |
| 09/12/2024 | **(10/12/2024)** |

| Name and mailing address of the ISA/ | Authorized officer |
| --- | --- |
| | J. Vizán Arroyo |
| OFICINA ESPAÑOLA DE PATENTES Y MARCAS Paseo de la Castellana, 75 - 28071 Madrid (España) Facsimile No.: 91 349 53 04 | Telephone No. 913498573 |

Form PCT/ISA/210 (second sheet) (July 2022)

## INTERNATIONAL SEARCH REPORT

| International application No. |
|---|
| PCT/ES2024/070483 |

### C (continuation). DOCUMENTS CONSIDERED TO BE RELEVANT

| Category * | Citation of documents, with indication, where appropriate, of the relevant passages | Relevant to claim No. |
|---|---|---|
| A | JUSZCZAK, A. et al. Plasma fucosylated glycans and C-reactive protein as biomarkers of HNF1A-MODY in young adult-onset nonautoimmune diabetes. Diabetes Care. Jan 2019, Vol. 42, No 1, pages 17 - 26, ISSN 1935-5548 (electronic), <DOI: 10.2337/dc18-0422> the whole document. | 1-8 |
| A | BELLANNE-CHANTELOT, C. et al. High-sensitivity C-reactive protein does not improve the differential diagnosis of HNF1A-MODY and familial young-onset type 2 diabetes: A grey zone analysis. Diabetes & Metabolism Feb 2016, Vol. 42, No 1, pages 33-37, ISSN 1262-3636 (print), ISSN 1878-1780 (electronic), <DOI: 10.1016/j.diabet.2015.02.001> the whole document. | 1-8 |
| A | BALTACI, O. F. et al. Exploring the role of miRNAs in the diagnosis of MODY3. Turkish Journal of Medical Sciences Turkey. Jun 2018, Vol. 48, No 3, pages 620 - 627, ISSN 1300-0144 (print), <DOI: 10.3906/sag-1711-98> the whole document. | 1-8 |

Form PCT/ISA/210 (continuation of second sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HE et al.** *Nat. Rev. Genet.*, July 2004, vol. 5 (7), 522-31
- **LIVAK et al.** *Methods*, 2001, vol. 25, 402-408
- **CATANZARO, G** ; **BESHARAT, Z. M.** ; **CHIAC-CHIARINI, M.** ; **ABBALLE, L.** ; **SABATO, C.** ; **VACCA, A** ; **FERRETTI, E**. Circulating MicroRNAs in elderly type 2 diabetic patients. *International Journal of Endocrinology*, 2018
- **CHIEN, H. Y** ; **LEE, T. P.** ; **CHEN, C. Y** ; **CHIU, Y. H.** ; **LIN, Y. C.** ; **LEE, L. S.** ; **LI, W. C.** Circulating microRNA as a diagnostic marker in populations with type 2 diabetes mellitus and diabetic complications.. *Journal of the Chinese Medical Association*, 2015, vol. 78
- **CORONA-MERAZ, F. I.** ; **VÁZQUEZ-DEL MERCA-DO, M** ; **ORTEGA, F. J.** ; **RUIZ-QUEZADA, S. L.** ; **GUZMÁN-ORNELAS, M. O.** ; **NAVARRO-HERNÁN-DEZ, R. E.** Ageing influences the relationship of circulating miR-33a and miR-33b levels with insulin resistance and adiposity. *Diabetes and Vascular Disease Research*, 2019, vol. 16 (3), 244-253
- **CANDIA, P** ; **TORRI, A** ; **PAGANI, M.** ; **ABRIGNANI, S**. Serum microRNAs as biomarkers of human lymphocyte activation in health and disease. *Frontiers in Immunology*, 2014, vol. 5
- **DP, B.** MicroRNAs : Genomics, Biogenesis, Mechanism, and Function.pdf. *Cell*, 2004
- **ESAU, C.** ; **DAVIS, S.** ; **MURRAY, S. F** ; **YU, X. X** ; **PANDEY, S. K** ; **PEAR, M.** ; **MONIA, B. P**. miR-122 regulation of lipid metabolism revealed by in vivo antisense targeting. *Cell Metabolism*, 2006, vol. 3 (2), 87-98
- **JUSZCZAK, A., PAVIĆ, T., VUČKOVIĆ, F** ; **BENNETT, A. J.** ; **SHAH, N** ; **MEDVIDOVIĆ, E. P.** ; **GORNIK, O.** Plasma fucosylated glycans and C-reactive protein as biomarkers of HNF1A-MODY in young adult-onset nonautoimmune diabetes. *Diabetes Care*, 2019, vol. 42 (1), 17-26
- **KATO, M** ; **CASTRO, N. E** ; **NATARAJAN, R.** MicroRNAs: Potential mediators and biomarkers of diabetic complications. *Free Radical Biology and Medicine*, 2013, vol. 64, 85-94
- **LA SALA, L** ; **MICHELONI, S.** ; **DE NIGRIS, V** ; **PRATTICHIZZO, F.** ; **CERIELLO, A.** Novel insights into the regulation of miRNA transcriptional control: implications for T2D and related complications. *Acta Diabetologica*, 01 October 2018, vol. 55, 989-998
- **NUNEZ LOPEZ, Y. O.** ; **RETNAKARAN, R.** ; **ZINMAN, B.** ; **PRATLEY, R. E.** ; **SEYHAN, A. A**. Predicting and understanding the response to short-term intensive insulin therapy in people with early type 2 diabetes. *Molecular Metabolism*, 2019, vol. 20, 63-78
- **POY, M. N.** ; **ELIASSON, L** ; **KRUTZFELDT, J.** ; **KUWAJIMA, S** ; **MA, X** ; **MACDONALD, P. E**. *Nature*, vol. 432 (7014), 226-230
- **PRATTICHIZZO, F.** ; **GIULIANI, A.** ; **DE NIGRIS, V** ; **PUJADAS, G** ; **CEKA, A** ; **LA SALA, L** ; **CERIELLO, A.** Extracellular microRNAs and endothelial hyperglycaemic memory: a therapeutic opportunity?. *Diabetes, Obesity and Metabolism*, 01 September 2016, vol. 18, 855-867
- **SPINETTI, G.** ; **FORTUNATO, O** ; **CAPORALI, A** ; **SHANTIKUMAR, S** ; **MARCHETTI, M** ; **MELONI, M**. *Circulation Research*, vol. 112 (2), 335-346
- **YANG, Z. M.** ; **CHEN, L. H.** ; **HONG, M** ; **CHEN, Y. Y** ; **YANG, X. R.** ; **TANG, S. M** ; **CHEN, W. W.** Serum microRNA profiling and bioinformatics analysis of patients with type 2 diabetes mellitus in a Chinese population. *Molecular Medicine Reports*, 2017, vol. 15, 4
- **ZAMPETAKI, A.** ; **KIECHL, S** ; **DROZDOV, I** ; **WILLEIT, P** ; **MAYR, U** ; **PROKOPI, M.** *Circulation Research*, vol. 107 (6), 810-817